# EUROPEAN PATENT SPECIFICATION

(11) **EP 3 979 967 B1**
(45) Date of publication and mention of the grant of the patent: **25.02.2026**
(21) Application number: 20819030.6
(22) Date of filing: 02.06.2020
(51) Int. Cl.: A61M 25/10, A61M 25/00

(54) **SYSTEMS FOR TREATING EDEMA**
SYSTEME ZUR BEHANDLUNG EINES ÖDEMS
SYSTÈMES DE TRAITEMENT D'OEDÈME

(30) Priority: 04.06.2019 US 201962857058 P
(43) Date of publication of application: 13.04.2022
(73) Proprietor: White Swell Medical Ltd, 6099000 Kibbutz Shefayim (IL)
(72) Inventor: NITZAN, Yaacov, 4636535 Hertzelia (IL); BRADY, Eamon, Loughrea Co. Galway, H62NX61 (IE)
(74) Representative: HGF
(86) International application number: PCT/IB2020/000419
(87) International publication number: WO 2020/245649

(56) References cited:
- EP-A1- 2 497 524
- WO-A1-2019/027380
- US-A1- 2006 178 604
- US-A1- 2010 280 451
- US-A1- 2010 280 451
- US-A1- 2014 336 551
- US-A1- 2015 051 634
- US-A1- 2018 185 622
- US-A1- 2019 126 014

## Description

### Technical Field

The disclosure relates to devices for the treatment of edema.

### Background

Congestive heart failure occurs when the heart is unable to pump sufficiently to maintain blood flow to meet the body's needs. A person suffering heart failure may experience shortness of breath, exhaustion, and swollen limbs. Heart failure is a common and potentially fatal condition. In 2015 it affected about 40 million people globally and around 2% of adults overall. As many as 10% of people over the age of 65 are susceptible to heart failure.

In heart failure, the pressures in the heart ventricles and atria are excessively elevated. As a result, the heart works harder to eject blood, leading to a buildup of blood pressure, which may result in edema forming within interstitial compartments of the body. Edema refers to the abnormal accumulation of fluid in tissues of the body and results when elevated blood pressure prevents lymphatic fluid from draining from the interstitium. The additional work of the heart, with time, weakens and remodels the heart thus further reducing the ability of the heart to function properly. The fluid accumulation leads to dyspnea and acute decompensated heart failure (ADHF) hospitalization. Those conditions may result in severe health consequences including death.

Some approaches to the treatment of edema have sought to use intravascular pumps to restore flow. However, it is late suspected that the introduction of a bio-incompatible and mechanically complex pump may be associated with thrombosis, clotting, or hemolysis. Such a response may impede blood flow or yield dislodged thrombus that threaten embolic stroke.

US 2010/0280451 reports an occlusion perfusion catheter with first and second occlusion balloons with a space-occupying balloon between the two occlusion balloons.

EP 2 497 524 A1 reports a multi-balloon catheter system in which a catheter carries first, second and third balloons in which an agent is delivered, via the middle balloon to a chamber created by inflating the outer balloons.

WO 2019/027380 reports a catheter having perforated balloons and perforations formed on the shaft which are formed between the inflated distal and proximal balloons that are inflated to provide dilatation through hydrodynamic pressure and vascular lesion target site contact.

### Summary

The invention is defined in independent claim 1. Further aspects are set out in dependent claims 2-7.

The invention provides devices that improve the flow of lymph without the use of a rotating mechanical pump. Systems of the disclosure use inflatable balloons or similar restriction devices to transiently create impediments to flow in the vena cava. The removal of those impediments promotes active transport of lymph out of a thoracic duct and into systemic circulation. By using inflatable balloons or similar devices, an active pump is created in the area of the venous angle that causes lymph to flow out of the thoracic duct. Natural one-way valves of the lymph system inhibits backflow from the veins into the lymphatic system. Additional episodes of transient impediment may be created and relieved, which encourages additional lymph flow. Using a device such as an inflatable balloon, a series of such episodes may be provided. For example, by causing about ten to twenty episodes of transient impediment and relief, lymph flow may be restored to healthy rates and volumes. By such means, a subject may be treated for edema or ADHF.

In certain aspects, the disclosure provides a method (not part of the claimed invention) of treating edema. The method includes providing one or more impediments to blood flow through a venous angle of a subject and removing the impediment(s) to thereby cause active transport of lymphatic fluid out of the thoracic duct and into systemic circulation. Providing the impediment may include inserting a catheter into the venous angle and inflating a balloon disposed along a segment of the catheter. The method may include inflating and deflating the balloon at least about ten times per minute for at least about a minute. In some embodiments, the inflating step is performed using a pump external to the subject and fluidically coupled to the balloon via an inflation lumen extending through the catheter. For rapid inflation & deflation, the inflation lumen should open to an interior of the balloon at a large skive in a side of the segment of the catheter. Preferably the skive is dimensioned to permit at least about twenty inflation and deflation cycles per minute of the balloon.

In other embodiments, providing the impediment includes implanting an expandable member in the subject, just outside of and adjacent to the venous angle, and expanding the expandable member to thereby restrict a cross-sectional area of a vein of the venous angle.

The impediment may be provided within an internal jugular vein, an external jugular vein, a subclavian vein, an innominate vein, or a combination thereof. The impediment may be provided by inserting a catheter, through an incision on or near a neck of the subject, into a jugular vein and operating a pump to inflate a balloon on a distal segment of the catheter, with the balloon being repeatedly inflated and deflated in the jugular vein, cranial to the output of the lymph duct. Preferably, the impediment is provided using an implant or balloon catheter, and no pump or rotating mechanical device is inserted into vasculature of the subject.

Aspects of the disclosure provide a treatment device that includes a balloon and a catheter comprising an inflation lumen in fluidic communication with the balloon via a skive large enough to allow for rapid inflation and deflation of the balloon multiple times per minute. Preferably, the skive is dimensioned to permit at least about twenty inflation and deflation cycles per minute of the balloon. The balloon may be about two to five cm in length. The device may include an inflation pump at a proximal end of the catheter and in fluidic communication with the inflation lumen. The device may further include a pressure sensor on a portion of the catheter distal to the balloon. The device may also include a controller subsystem operable to receive a pressure reading from the pressure sensor and issue instructions to operate the pump.

In some embodiments, the controller subsystem inflates and deflates the balloon so that the balloon is inflated for about one hundred to about five hundred ms. For example, the controller system may inflate the balloon for brief pulses to decrease pressure in the venous angle to at least about fifty percent of a baseline pressure in the venous angle. The inflation lumen may have a cross-sectional area of at least about half of a cross-sectional area of the catheter.

The device may include a second balloon disposed along a segment of the catheter, such that the balloon and the second balloon can be positioned in a venous angle of a subject, upstream and downstream, respectively, of an outlet of a lymph duct. Preferably, the catheter extends between the balloon and the second balloon smoothly and continually, with no ports, outlets, for features.

The invention provides a system for treating edema. The system includes a plurality of occlusion balloons configured to occlude a plurality of veins upstream and downstream of a lymphatic duct so as to isolate a volume of space in the region of the lymphatic duct; a first catheter connected at least a first one of the occlusion balloons; at least one volumetric balloon disposed along the catheter and configured occupy a substantial portion of the volume of space; and at least one inflation lumen operable to inflate and deflate the plurality of balloons. In certain two-catheter embodiments, the catheter carries at least one of the occlusion balloons and the system also includes a second catheter bearing a second occlusion balloon and a second volumetric balloon. The first catheter includes the first occlusion balloon and a third occlusion balloon.

The catheter is dimensioned for insertion into a jugular vein and the second catheter is dimensioned for insertion into a subclavian vein. When the catheter and the second catheter are so inserted and the first, second, and third occlusion balloons are inflated, the occlusion balloons sequester the volume of space about the region of the lymphatic duct. Additionally, when the occlusion balloons are inflated, the first and second volumetric balloons can be inflated to displace blood from the volume of space. Moreover, when the volumetric balloons are deflated, the volume of space is depressurized, which urges lymph through a valve outlet of the lymphatic duct.

In preferred embodiments, the at least one volumetric balloon is configured to collapse to a relatively smaller volume and, when so collapsed, effects a region of reduced pressure between said occlusion balloons. The reduced pressure causes a flow of lymphatic fluid into the region of depressurization.

In the system, the occlusion balloons may be configured to be inflated and deflated cyclically. Preferably, when the system is in an inflated configuration each balloon occludes its upstream or downstream vessel. The upstream vessels may include one or more of an internal jugular vein, subclavian vein, or external jugular vein, and the downstream vessels may include the innominate vein and or the superior vena cava. The system may also have a control mechanism to control the cycle of inflation and deflation.

Certain aspects of the disclosure provide a method (not part of the claimed invention) of treating edema. The method preferably includes using a system comprising at least one occlusion balloon and at least one volumetric balloon to inhibit blood flow through, and substantially exclude blood from, a region of a venous angle, and deflating at least the volumetric balloon to cause active transport of lymphatic fluid out of the thoracic duct and into systemic circulation. Embodiments of the method include inserting a first catheter comprising at least a first collusion balloon into an internal jugular vein, inserting a second catheter comprising a second occlusion balloon into a subclavian or brachial vein, inflating the occlusion balloons and at least one volumetric balloon, volumetrically displacing blood (via the volumetric balloon) between the occlusion balloons in advance of sealing engagement of the occlusion balloons, and deflating the volumetric balloon while maintaining sealing engagement with the occlusion balloons. The method may further include holding at least a partial vacuum on the volumetric balloon for a period so as to depressurize the lymphatic outflow, collapsing the occlusion balloons while maintaining the position of the first and second catheters, and allowing a second period to elapse with the balloons collapsed. Preferably the method further repeating the steps for the duration of therapy.

The method may include removing the first and second catheter from the patient after the therapy duration has expired. Optionally, the step of inflating the occlusion balloons and the volumetric balloon involves inflating the first occlusion balloon in the internal jugular vein, inflating the second occlusion balloon in the subclavian vein, and inflating a third occlusion balloon in the innominate vein. The step of volumetrically displacing blood between the occlusion balloons may involve inflating at least one volumetric balloon before or at the same time as the occlusion balloons. The method thus allows normal or healthy flow to be restored to the region of the venous angle.

The methods of use and/or treatment described herein do not form part of the claimed invention and are left for illustrative purposes.

### Brief Description of the Drawings

FIG. 1 diagrams a method of treating edema.
FIG. 2 shows a treatment device for treating edema.
FIG. 3 shows a two-catheter system for treating edema.
FIG. 4 diagrams a method of treating edema using the two-catheter system.
FIG. 5 shows displacing blood from the venous angle with the two-catheter system.
FIG. 6 shows deflating volumetric balloons to restore flow.
FIG. 7 illustrates collapsing the two catheter system.
FIG. 8 diagrams an extravascular device useful for embodiments of the disclosure.
FIG. 9 shows detail of the extravascular device.
FIG. 10 shows a multi-lumen catheter system.
FIG. 11 shows the multi-lumen catheter system inflated.
FIG. 12 illustrates a final step in the use of the multi-lumen catheter system.
FIG. 13 shows a treatment catheter with exit ports.
FIG. 14 illustrates a system with console and manifold.

### Detailed Description

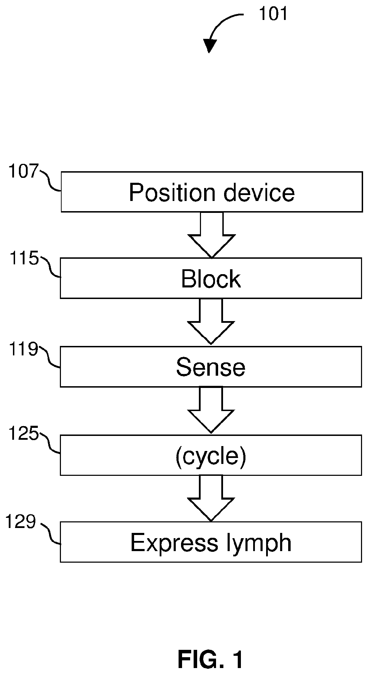

FIG. 1 diagrams a method 101 of treating edema. The method 101 includes positioning 107 a device within a subject, for example, within a venous angle. The device may be an intravascular catheter with a flow restrictor. The method includes providing an impediment, or multiple impediments, to blood flow through a venous angle of a subject to temporarily raise blood pressure and removing the impediment(s), thereby creating a transient decrease in blood pressure at an output of a lymph duct. For example, providing the impediment may be done by inserting a catheter into the venous angle and inflating a balloon disposed along a segment of the catheter. Preferably, the balloon is inflated via an inflation lumen along the catheter. The inflation lumen may open to an interior of the balloon at a skive in a side of the segment of the catheter and the skive is cut large enough to permit rapid inflation and deflation of the balloon, to provide the transient impediments.

The device is used to block 115 flow through a vein. Accordingly, the device provides an impediment to blood flow through a venous angle of a subject to temporarily raise blood pressure above a baseline cranial to the impediment. The impediment is removed, which results in a transient decrease in blood pressure at an output of a lymph duct. In response, the body expresses 129 lymph from the lymph duct and into the venous angle.

It may be most preferable to perform the method 101 by creating a cycle 125, or repeated set, of the transient impediments. For example, the method 101 may include inflating and deflating the balloon at least about ten times per minute for at least about a minute. Steps of the method 101 may be performed using systems or devices of the disclosure.

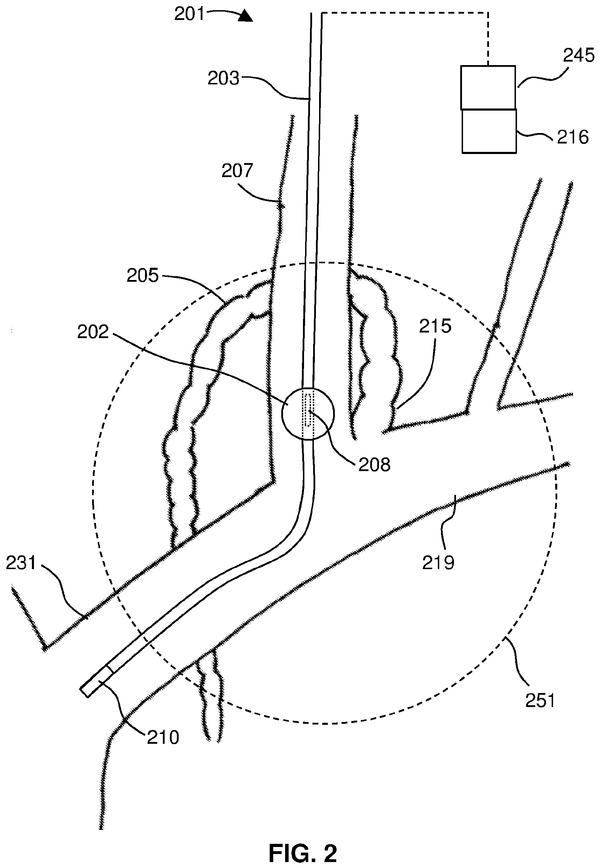

FIG. 2 shows a treatment device 201 for treating edema. The device 201 includes a balloon 201 and a catheter comprising an inflation lumen in fluidic communication with the balloon via a skive 208 (e.g., on a side of the catheter on an inside of the balloon). The skive 208 comprises a cut through the catheter that is large enough to allow for rapid inflation and deflation of the balloon multiple times per minute. Preferably, the skive 208 is dimensioned to permit at least about twenty inflation and deflation cycles per minute of the balloon. The balloon 202 may be about two to five cm in length. The device 201 may include an inflation pump 245 at a proximal end of the catheter and in fluidic communication with the inflation lumen. The device 201 may further include a pressure sensor 210 on a portion of the catheter distal to the balloon. The device 201 may include a controller subsystem 216 operable to receive a pressure reading from the pressure sensor 210 and issue instructions to operate the pump 245.

The device includes a pump 245 such as a programmable pump that can control inflation of, and time-varying patterns of inflation of, the balloon 202. A volume of the balloon 202 may be controlled by a pump 245 (e.g., an oscillating pump) in a closed-air system to allow rapid bidirectional volume transfer. In some embodiments, motion of the pump piston is controlled by a control subsystem 216 (e.g., running on a computer system). Optionally, based on input from a pressure sensor 210, the control subsystem synchronizes the forward and backward motion of the piston in the pump 245 to apply a series of transient impediments to flow.

Any suitable pump may be used. For example, in some embodiments, the pump has a 24 mm diameter, 50 mm stroke length air-filled antifriction cylinder, e.g., as available from Airpot Corp (Norwalk, CT), with a piston driven by a stepper motor using a ball screw linear actuator (Model EZC6-05, Oriental Motors Co., Ltd), via a ball-joint interconnection. The stepper motor is, in turn, controlled and driven by a dedicated motor controller/driver, e.g., as available from Oriental Motor U.S.A. Corp. (Torrance, CA).

The control subsystem 216 may be provided by a computer system that includes at least one processor connected to memory and a set of input/output (I/O) devices.

The device 201 is useful to perform steps of the method 101. For example, in the method 101, the inflating step may be performed using the pump external 245 to the subject and fluidically coupled to the balloon 202 via an inflation lumen extending through the catheter 203. The skive 208 is dimensioned to permit at least about twenty inflation and deflation cycles per minute of the balloon 202.

The device 201 may be inserted into the venous angle 251. As shown, a distal segment of the catheter 203 sits in an innominate vein 231, having been extended through an internal jugular vein 211. The balloon 202 sits near a terminal lymphangion 215 of a lymphatic duct 205, nearby a junction with the subclavian vein 219. Using the device 201 for the method 101, the impediment is preferably provided within an internal jugular vein 211, 207, an external jugular vein, a subclavian vein 219, an innominate vein 231, or a combination thereof.

The impediment may be provided by inserting the catheter 203 through an incision on or near a neck of the subject, through the jugular vein 211, and operating the pump 245 to inflate the balloon 202, which is on a distal segment of the catheter 203. Preferably, the balloon 202 is repeatedly inflated and deflated in the jugular vein 211, cranial to the output of the lymph duct 205. As shown, the impediment is provided using an implant or balloon catheter, and no pump or rotating mechanical device is inserted into vasculature of the subject. This avoids any risk of pump thrombosis, as there is no mechanical pump inside any blood vessel. The controller subsystem 216 may be operated to inflate and deflate the balloon so that the balloon is inflated for about one hundred to about five hundred ms. Using the pressure sensor 210, the controller system may inflate the balloon for brief pulses to decrease pressure in the venous angle to at least about fifty percent of a baseline pressure in the venous angle. Preferably, the inflation lumen is large, e.g., has a cross-sectional area of at least about half of a cross-sectional area of the catheter. The device 201 may optionally include a second balloon disposed along a segment of the catheter, such that the balloon and the second balloon can be positioned in a venous angle of a subject, upstream and downstream, respectively, of an outlet of a lymph duct. Preferably, the catheter extends between the balloon and the second balloon smoothly and continually, with no ports, outlets, for features.

The method 101 and the device 201 are useful for draining lymph or treating edema and do not require the provision of an intravascular mechanical part such as a rotary pump and thereby avoid a risk of pump thrombosis. Other embodiments are within the scope of the disclosure and include methods and systems by which multiple balloons are used to promote lymph drainage, also within any intravascular rotating pump. For example, in some embodiments, a system of catheters is used to create flow blockages that exploit principles of flow and fluid dynamics and the one-way valves that naturally define a lymphatic duct to cause lymph to flow and drain from the lymphatic system.

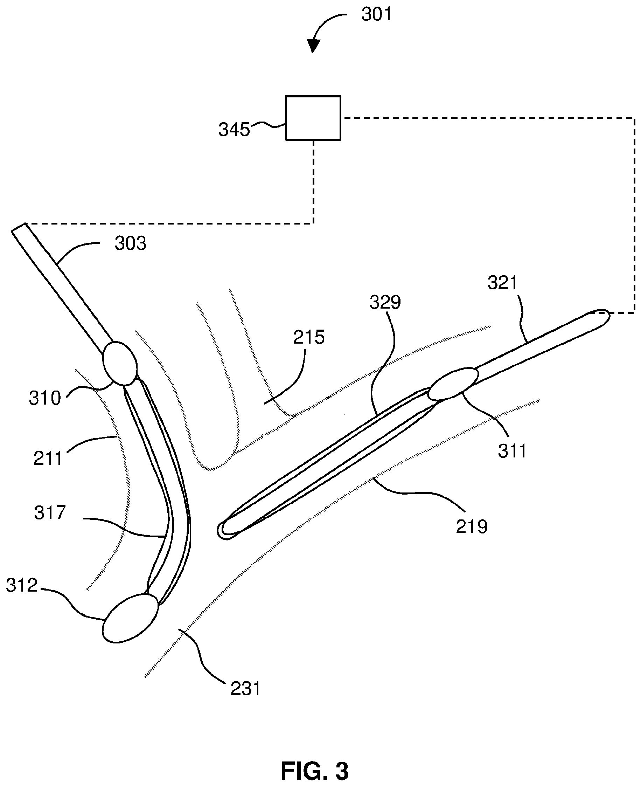

FIG. 3 shows a system 301 for treating edema. The system 301 includes a plurality of occlusion balloons 310, 311, 312 configured to occlude a plurality of veins upstream and downstream of a lymphatic duct 215 so as to isolate a volume of space in the region of the lymphatic duct. The system 301 includes at least one catheter 303 connected to at least a first occlusion balloon 310 and at least one volumetric balloon 317 disposed along the catheter and configured to occupy a substantial portion of the volume of space. An inflation lumen extends through the catheter 303 and is operable to inflate and deflate the first occlusion balloon 310. In the depicted embodiment, the catheter 303 carries the first occlusion balloon 310 and the system 301 also includes a second catheter 321 bearing: a second occlusion balloon 311 and a second volumetric balloon 329. Moreover, in the depicted embodiment, the catheter 303 includes the first occlusion balloon 310 and a third occlusion balloon 312.

Preferably, the catheter 303 is dimensioned for insertion into a jugular vein 211 and the second catheter 321 is dimensioned for insertion into a subclavian vein 219. When the catheter and the second catheter are so inserted and the first, second, and third occlusion balloons 310, 311, 312 are inflated, the occlusion balloons sequester the volume of space about the region of the lymphatic duct 215. Moreover, when the occlusion balloons 310, 311, 312 are inflated, the first and second volumetric balloons 317, 329 can be inflated to displace blood from the volume of space. When the volumetric balloons 317, 329 are deflated, the volume of space is depressurized, which urges lymph through a valve outlet of the lymphatic duct. Thus the system uses at least one volumetric balloon that is configured to collapse to a relatively smaller volume and, when so collapsed, effects a region of reduced pressure between the occlusion balloons, said reduced pressure causing a flow of lymphatic fluid into the region of depressurization.

The system 301 is useful in a method for treating edema.

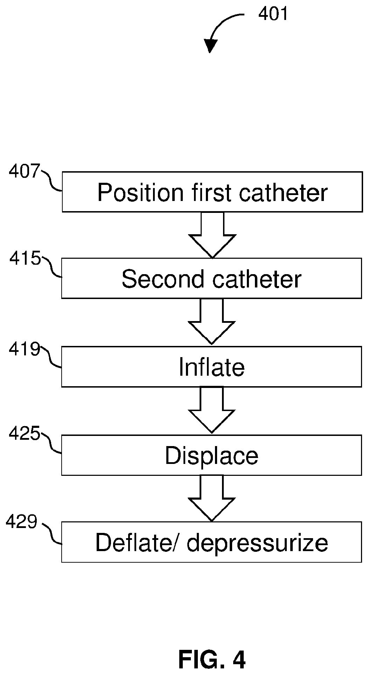

FIG. 4 diagrams a method 401 of treating edema. The method 401 includes inserting 401 a first catheter comprising at least a first collusion balloon into an internal jugular vein, inserting 415 a second catheter comprising a second occlusion balloon into a subclavian or brachial vein, inflating 419 the occlusion balloons and at least one volumetric balloon and volumetrically displacing 425 blood (via the volumetric balloon) between the occlusion balloons in advance of sealing engagement of the occlusion balloons. Further, the method 401 includes deflating 429 the volumetric balloon while maintaining sealing engagement with the occlusion balloons, holding at least a partial vacuum on the volumetric balloon for a period so as to depressurize the lymphatic outflow, collapsing the occlusion balloons while maintaining the position of the first and second catheters, and allowing a second period to elapse with the balloons collapsed,

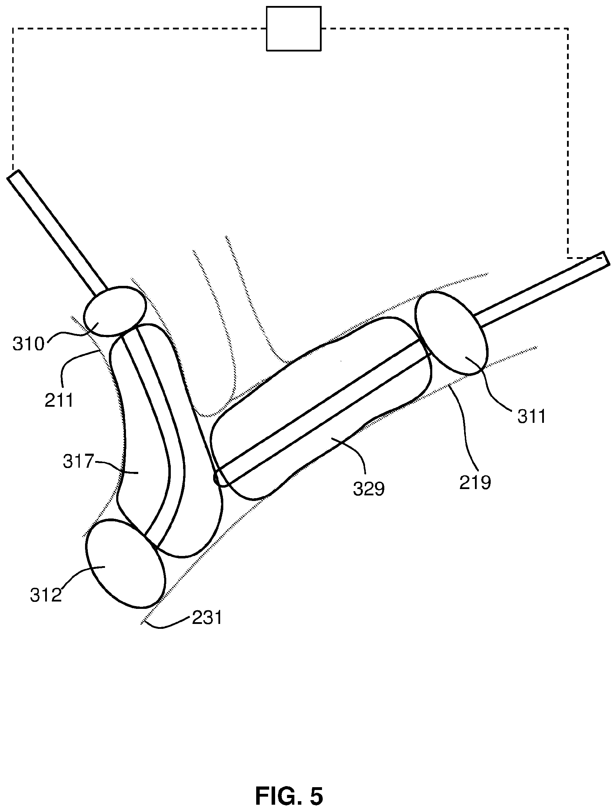

FIG. 5 shows the step of volumetrically displacing 425 blood from the venous angle. While there may be some permissiveness in the exact order of the steps, essentially the volumetric balloons 317, 329 are inflated and so are the occlusion balloons 310, 311, 312. Those inflations essentially exclude substantially all blood from the area of the venous angle relevant to the outlet of the thoracic duct 215. The next step of the method 401 is to deflate the volumetric balloons 317, 329.

As shown, embodiments of the method 401 include-for the step of inflating the occlusion balloons and the volumetric balloon-inflating the first occlusion balloon 310 in the internal jugular vein 211, inflating the second occlusion balloon 311 in the subclavian vein 219, and inflating a third occlusion 312 balloon in the innominate vein 231. The step of volumetrically displacing blood between the occlusion balloons may include inflating one or both of the volumetric balloons 317, 329 before or at the same time as the occlusion balloons.

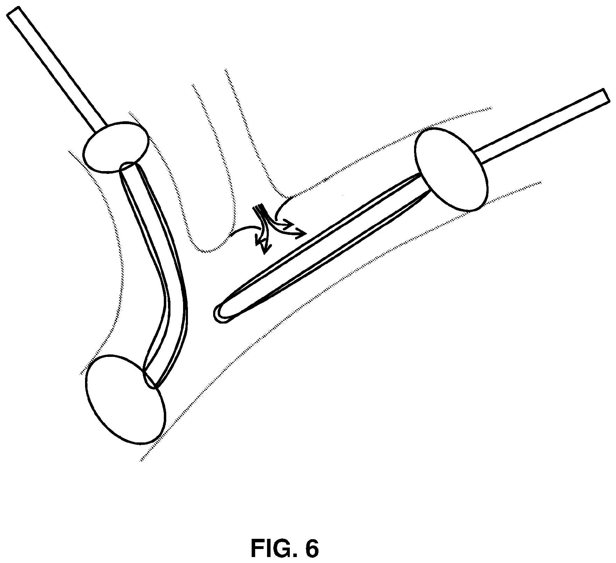

FIG. 6 illustrates the results of deflating the volumetric balloons 317, 329. In the system 301, the balloons are preferably configured to be inflated and deflated cyclically. When the system is in an inflated configuration each occlusion balloon occludes its upstream or downstream vessel. The upstream vessels may include one or more of an internal jugular vein, subclavian vein, or external jugular vein, and the downstream vessels may include the innominate vein and or the superior vena cava. As shown, when the volumetric balloons 317, 329 are deflated while maintaining sealing engagement with the occlusion balloons 310, 311, 312. At least a partial vacuum may be held on the volumetric balloons 317, 329 for a period so as to depressurize the lymphatic outflow, promoting lymph flow. Lymph flows out of the thoracic duct 215. Thus the method 401 includes allowing normal flow to be restored to the region of the venous angle.

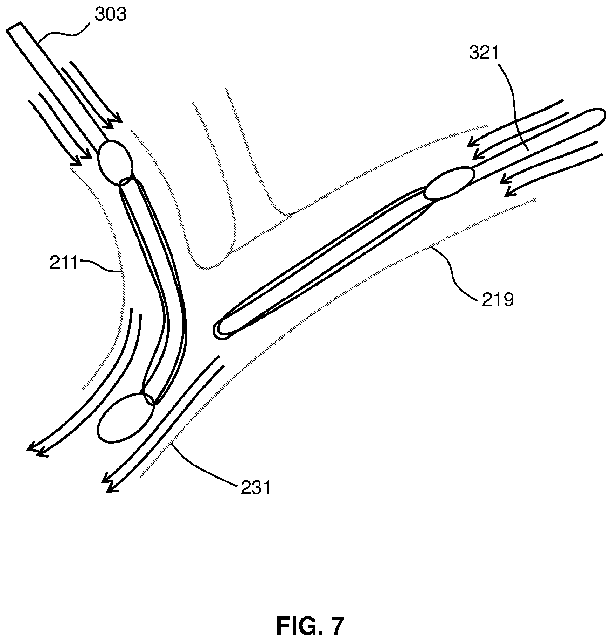

FIG. 7 illustrates collapsing the occlusion balloons 310, 311, 312 while maintaining the position of the first and second catheters, and allowing a second period to elapse with the balloons collapsed. Normal blood flow is restored through the internal jugular vein 211, the subclavian vein 219, and the innominate vein 231. The system 301 may be operated under the control of a control mechanism to control the cycle of inflation and deflation. The method 401 may include repeating the steps for the duration of therapy.

Finally, the method 401 may include removing the first catheter 303 and the second catheter 321 from the patient after the therapy duration has expired.

Certain embodiments of the disclosure are directed to intravascular methods, devices, and systems for the treatment of edema. However, the disclosure encompasses any method, device, or system for treating edema that involves providing an impediment to blood flow through a venous angle of a subject to temporarily raise blood pressure above a baseline cranial to the impediment and removing the impediment, to thereby create a transient decrease in blood pressure at an output of a lymph duct. Some aspects and embodiments of the disclosure perform such steps without an intravascular device, using instead a subcutaneous implant or an extracorporeal device. In fact, the disclosure includes the use of both intravascular and extravascular intervention devices and methods in combination. An exemplary extravascular edema treatment device is shown and discussed.

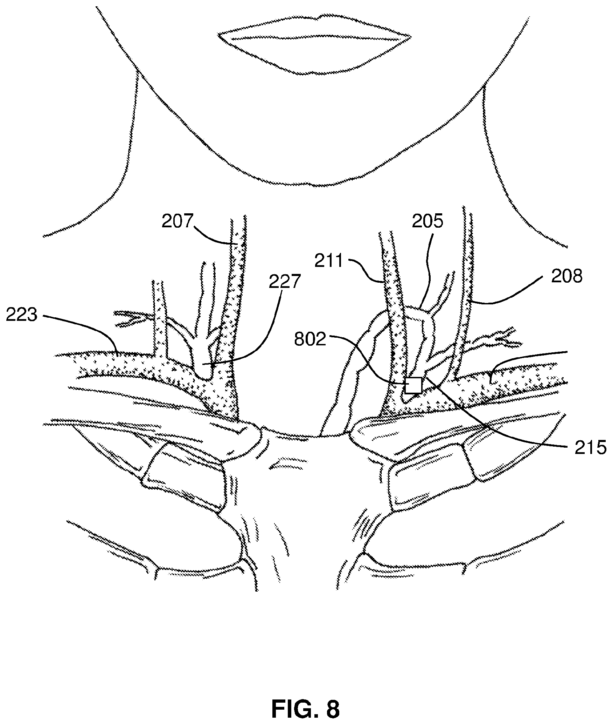

FIG. 8 diagrams a device useful for other embodiments of methods of the disclosure. The device makes use of an implantable device 802 that can be operated to provide an impediment to flow through a vein of the venous angle. As shown in the figure, the device 802 is implanted immediately outside of and adjacent an internal jugular vein 211. Any suitable implant may be used for the device 802. For example, the device 802 may be a magnet and operating it may be performed by bringing an extracorporeal second magnet into proximity of the subject, with like poles facing each other. The force of the external magnet on the magnet device 802 causes the device 802 to compress the internal jugular vein 211, which impedes blood flow into the venous angle. The impediment to flow is then removed (by moving the external second magnet away from the body) and blood rushes to flow through the internal jugular vein. The rush of blood through internal jugular vein has a velocity higher than normal, which, by the principle of Bernoulli, implies a decreased lateral pressure. Thus a relatively low pressure is exerted against the one-way outlet valve at a terminal lymphangion of the thoracic duct. Due to this decreased pressure, lymph flows from the terminal lymphangion and into the venous angle. A series of such impediments may be created and relived (e.g., by bringing the external second magnet towards and away from the body repeatedly) which effectively creates a pumping action, pumping lymph from the thoracic duct.

Other embodiments are within the scope of the disclosure. For example, in some embodiments, the device 201 is provided as an expandable member implanted into the subject adjacent a vein of the venous angle and connected, via a catheter with an inflation lumen, to an external pump. Accordingly, in some embodiments of the method 101, providing the impediment can be done by implanting an expandable member 802 in the subject, just outside of and adjacent a vein of the venous angle, and expanding the expandable member to thereby restrict a cross-sectional area of a vein of the venous angle.

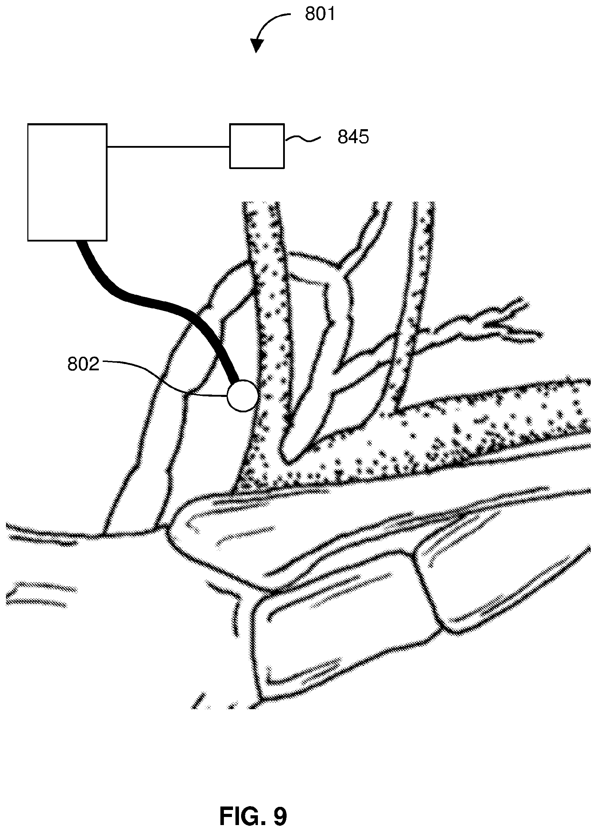

FIG. 9 shows detail of the device 801 with the expandable member 802. The device 801 includes an expandable member 802, such as a non-compliant or semi-compliant balloon. The member 802 is connected via a catheter 803 to a pump 845. The catheter 803 defines an inflation lumen fluidically coupling the pump 845 to the expandable member 802. The inflation lumen opens into the expandable member 802 via a skive cut large to allow for rapid inflation and deflation. The pump may optionally be under the control of a controller subsystem 816 that applies a logic or set of rules to operate the pump to cause the expandable member 802 to expand to create a series of transient impediments to flow through a vein (e.g., an internal jugular vein 211, a subclavian vein, an innominate vein) of the venous angle. Each transient impediment may be short-lived, e.g., about 100 to about 500 milliseconds. Such a series of impediments may be created about ten to about 20 times more minute or more.

Such a mechanical creation of impediments to flow establishes a series of pressure depressions associated (by Bernoulli) with velocity increases when blood flow is restored. Each pressure depression in the venous angle promotes the flow of lymph down a pressure gradient, out of the thoracic duct and into the venous angle. Thus, without using any intravascular mechanical pump, lymph flow can be promoted and restored, which is useful to treat congestive heart failure or edema. Not using an intravascular device with complex mechanical parts avoids a risk of hemolysis, clotting, or pump thrombosis. This avoid issues described in Blitz, 2014, Pump thrombosis- a riddle wrapped in a mystery inside an enigma, Ann Cardiothorac Surg 3(5):450-471 and Tchantchaleishvili, 2014, Evaluation and treatment of pump thrombosis and hemolysis, Ann Cardiothorac Surg 3(5):490-495.

The disclosure generally relates to devices and methods for the treatment of edema. Details may be found in Chikly, 2005, Manual techniques addressing the lymphatic system: origins and development, JAOA 105(10):457-464; Ratnayake, 2018, The anatomy and physiology of the terminal thoracic duct and ostial valve in health and disease: potential implications for intervention, J Anat 233:1-14; and U.S. Pub. 2016/0166463 A1. Other embodiments are within the scope of the disclosure. For example, some embodiments use a single catheter. Any embodiment may include any one or more of any of the following features in any combination: guidewire lumen, guidewire, exit ports, flow manifold, and control console.

In general, methods, devices, and systems of the disclosure embody a concept that uses an arrangement of balloons to positively pump (suck and release) fluid from the thoracic duct.

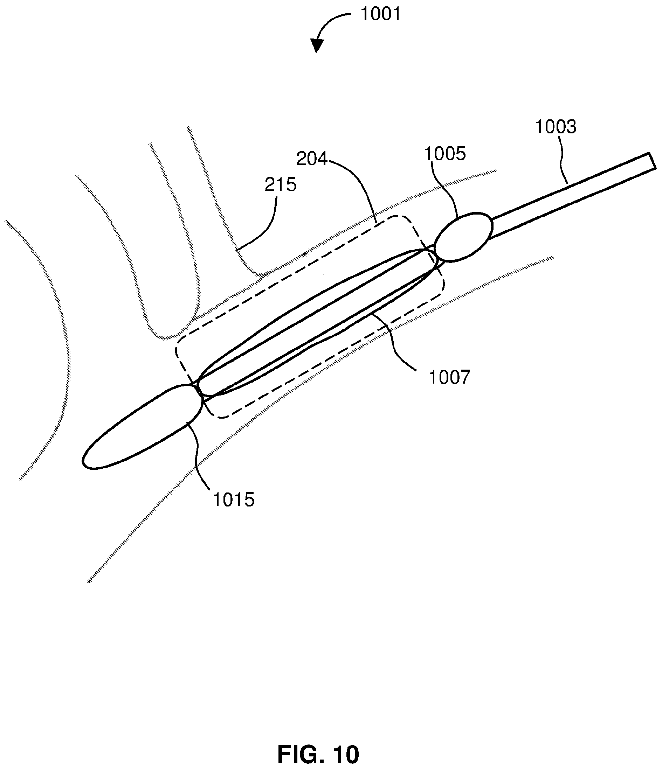

FIG. 10 shows a multi-lumen catheter system 1001 that includes three balloons. The system 1001 includes a proximal occlusion balloon 1005 and a distal occlusion balloon 1015, which allow a physician to isolate a volume 204 adjacent to the thoracic duct 215. The proximal occlusion balloon 1005 and a distal occlusion balloon 1015 are shown in a collapsed configuration. The proximal occlusion balloon 1005 and a distal occlusion balloon 1015 are carried by and supported upon a multi-lumen catheter 1003. The system includes a volumetric balloon 1007 that can be inflated to substantially fill the isolated volume 2004 adjacent to the thoracic duct 215.

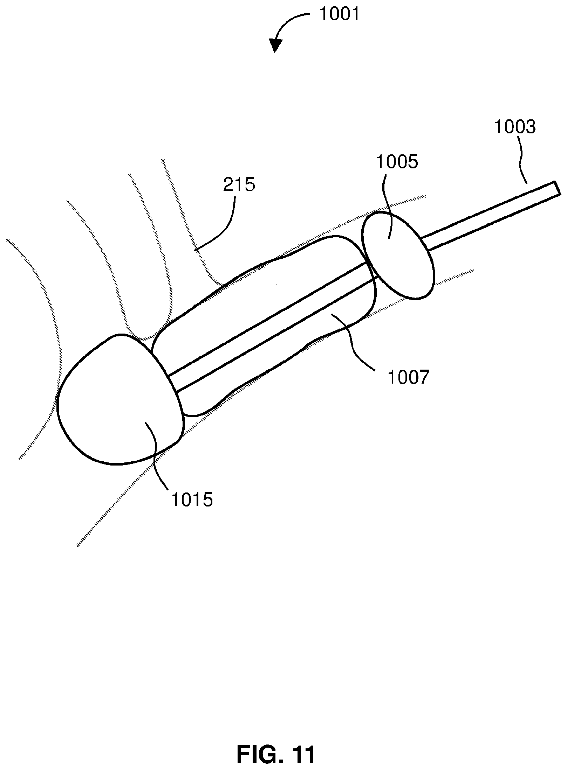

FIG. 11 shows the system 1001 inflated. The single proximal occlusion balloon 1005 is inflated and occludes the upstream vein (which may be the internal jugular vein or the subclavian vein). The distal occlusion balloon 1015 simultaneously occludes the Innominate, Distal internal jugular, and distal subclavian veins. The volumetric balloon 1007 is inflated simultaneous with the proximal and distal occlusion balloons. Blood is largely displaced from the isolated region.

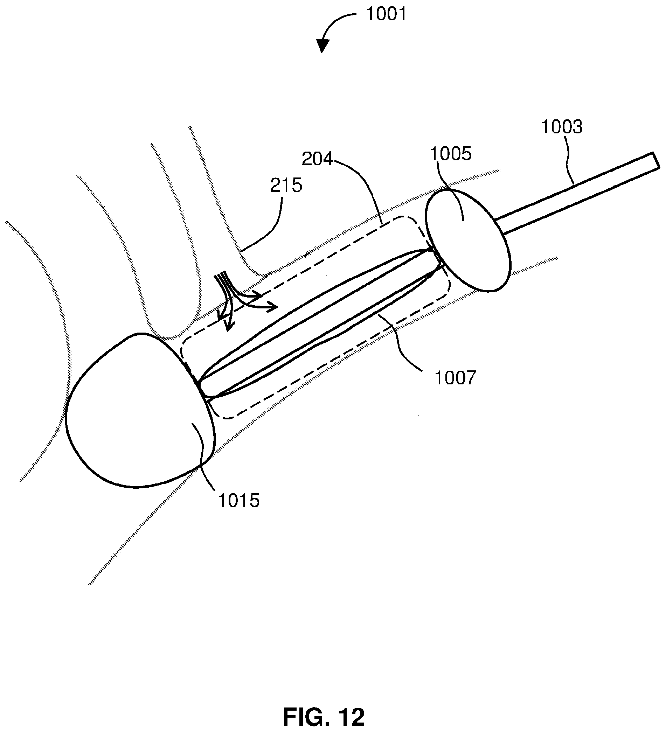

FIG. 12 illustrates a final step in the use of the system 1001. The proximal occlusion balloon 1005 and the distal occlusion balloon 1015 remain inflated. The volumetric balloon 1007 is deflated and causes fluid to flow from the thoracic duct 215 into the isolated volume 204.

Other embodiments and techniques are within the scope of the disclosure.

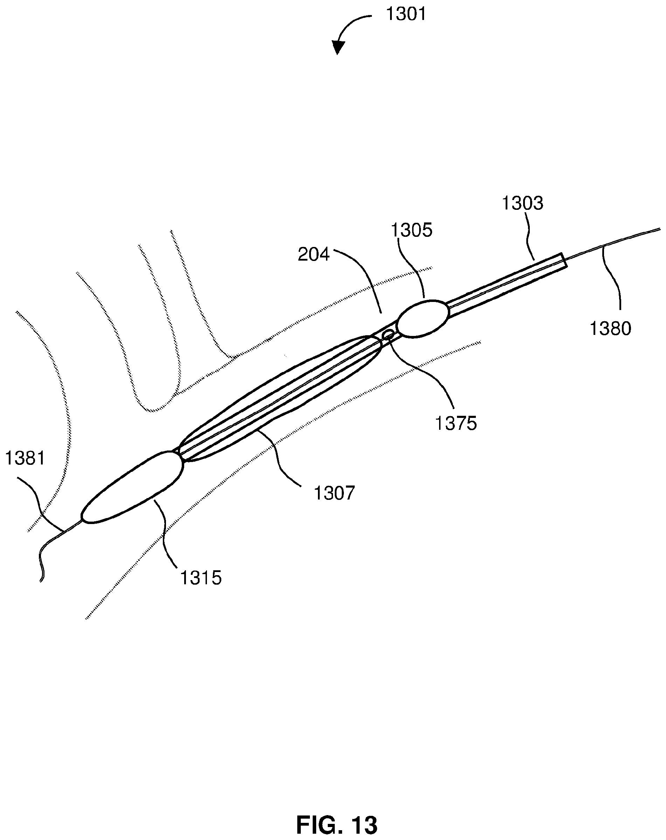

FIG. 13 shows a treatment catheter 1301 with one or more exit ports 1375, and illustrates the use of the exit ports 1375. The catheter 1301 sits on a guidewire with a proximal guidewire end 1380 and a distal guidewire end 3181. As shown, the catheter includes proximal occlusion balloon 1305 (collapsed) and a distal occlusion balloon 1315 (also collapsed). Between the proximal occlusion balloon 1305 and the distal occlusion balloon 1315 sits a volumetric balloon 1307, shown in a deflated conformation. An exit port 1375 is shown. The exit port 1375 is connected to a lumen in the catheter 1301 and it allows a doctor to take sample of lymph fluid contained in the isolation volume 204.

Embodiments of the disclosure include console and manifold variations.

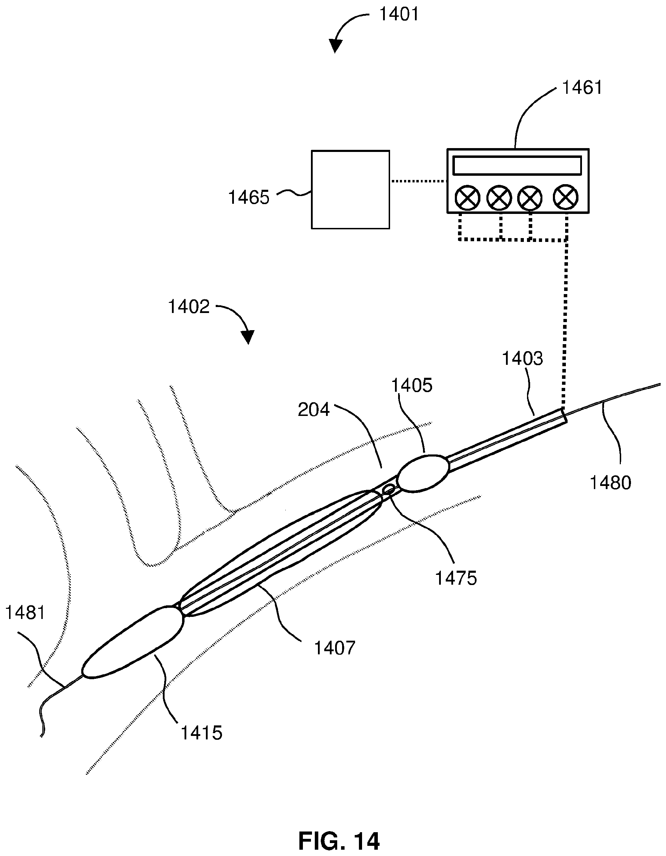

FIG. 14 illustrates a system 1401 for treating edema that includes a console 1465 and a manifold 1461 connected to a catheter 1301 of the disclosure. The catheter 1401 preferably includes exit ports 1475. The catheter 1401 sits on a guidewire with a proximal guidewire end 1480 and a distal guidewire end 1481. As shown, the catheter includes proximal occlusion balloon 1405 (collapsed) and a distal occlusion balloon 1415 (also collapsed). Between the proximal occlusion balloon 1405 and the distal occlusion balloon 1415 sits a volumetric balloon 1407, shown in a deflated conformation. An exit port 1475 is shown. The optional exit port 1475 is connected to a lumen in the catheter 1401 and allows a doctor to take sample of lymph fluid contained in the isolation volume 204.

An inflation manifold 1461 is provided to direct flow to the correct balloons according to methods of the disclosure. A console 1465 controls the cycle. Systems or methods of the disclosure may use either or both of an inflation manifold 1461 and a console 1465. The manifold 1461 is useful in any embodiments shown herein such as, for example, any embodiment with multiple balloons. Under control of any console 1465 or computer subsystem, the manifold 1461 cam control the series of inflation and deflation activities to cause the balloons (e.g., any included occlusion balloons or volumetric balloons) to exhibit the described patterns of inflated and collapsed/ deflated states.

Variations of a single catheter thoracic duct pump concept are within the scope of the disclosure. In certain embodiments, catheters are provided including an exit port for (a) sampling lymph fluid or blood, (b) pressure elements to measure pressure on the isolation region and CVP. Catheters according to various embodiments of the disclosure may also have a guidewire lumen for delivery.

The systems herein provide methods of treating edema. For example, using the catheter 1401 one perform a method of treating edema, in which the method includes: (a) isolating the venous angle and a lymphatic duct with at least two occlusion balloons, (b) simultaneously inflating at least one volumetric balloon between the at least two occlusion balloons, (c) urging fluid flow from the lymphatic duct into the venous angle, (d) deflating the occlusion balloons to allow lymph fluid trapped in the isolated region into the venous system, (e) allowing a time period to elapse, and (f) repeating steps (a) to (e) until therapy is complete. In preferred embodiments of this method, the step of (c) urging fluid flow comprises the step of deflating the volumetric balloon while maintaining isolation of the venous angle and the lymphatic duct with the at least two occlusion balloons. These methods steps are illustrated by FIGs. 10 through 13 and also separately by FIGs. 3, 5, 6, and 7. This disclosed method as shown in FIGs. 10 through 13 allows one to effect a thoracic duct pump with a single balloon catheter 1301. One key benefit of such a system and method, illustrated in FIG. 11, is that the distal balloon 1015 simultaneously occludes the three vessels of the venous angle when inflated (the IJ, IN and SV).

### Equivalents

Various modifications of the invention and many further embodiments thereof, in addition to those shown and described herein, will become apparent to those skilled in the art from the full contents of this document, including references to the scientific and patent literature cited herein.

## Claims

1. A system (301) for treating edema, the system (301) comprising:
a plurality of occlusion balloons (310, 311, 312) configured to occlude a plurality of veins upstream and downstream of a lymphatic duct (215) so as to isolate a volume of space in the region of the lymphatic duct (215);
a first catheter (303) connected to at least a first one of the occlusion balloons (310);
a first volumetric balloon (317) disposed along the first catheter (303) and configured occupy a substantial portion of the volume of space; and
at least one inflation lumen operable to inflate and deflate the plurality of balloons (310, 311, 312);
**characterised in that** the system (301) further comprises a second catheter (321), bearing: a second occlusion balloon (311), and a second volumetric balloon (329), and
wherein the first catheter (303) further comprises a third occlusion balloon (312) and further wherein the first catheter (303) is dimensioned for insertion into a jugular vein (211) and the second catheter (321) is dimensioned for insertion into a subclavian vein (219), and
wherein when the first catheter (303) and the second catheter (321) are so inserted and the first, second, and third occlusion balloons (310, 311, 312) are inflated, the occlusion balloons (310, 311, 312) sequester the volume of space about the region of the lymphatic duct (215), and
wherein when the occlusion balloons (310, 311, 312) are inflated, the first and second volumetric balloons (317, 329) can be inflated to displace blood from the volume of space.

2. The system (301) of claim 1, wherein when the first and second volumetric balloons (317, 329) are deflated, the volume of space is depressurized, which urges lymph through a valve outlet of the lymphatic duct (215).

3. The system (301) of claim 1, wherein at least one of the first or second volumetric balloons (317, 329) is configured to collapse to a relative smaller volume and, when so collapsed, effects a region of reduced pressure between said occlusion balloons (310, 311, 312), said reduced pressure causing a flow of lymphatic fluid into the region of depressurization.

4. The system (301) of claim 1, wherein the occlusion balloons (310, 311, 312) are configured to be inflated and deflated cyclically.

5. The system (301) of claim 1, wherein when the system (301) is in an inflated configuration each balloon (310, 311, 312) occludes its upstream or downstream vessel.

6. The system (301) of claim 5, wherein
the upstream vessels comprise one or more of an internal jugular vein, subclavian vein, or external jugular vein, and
the downstream vessels comprise the innominate vein and or the superior vena cava.

7. The system of claim 6, further comprising a control mechanism to control the cycle of inflation and deflation.

## Patentansprüche

1. System (301) zur Behandlung eines Ödems, wobei das System (301) umfasst:
eine Vielzahl von Okklusionsballons (310, 311, 312), die dazu konfiguriert sind, eine Vielzahl von Venen stromaufwärts und stromabwärts eines Lymphgangs (215) zu okkludieren, um so ein Raumvolumen in der Region des Lymphgangs (215) zu isolieren;
einen ersten Katheter (303), der mit mindestens einem ersten der Okklusionsballons (310) verbunden ist;
einen ersten volumetrischen Ballon (317), der entlang des ersten Katheters (303) angeordnet und dazu konfiguriert ist, einen wesentlichen Teil des Raumvolumens einzunehmen; und
mindestens ein Aufblaslumen, das betreibbar ist, um die Vielzahl von Ballons (310, 311, 312) aufzublasen und zu entleeren;
**dadurch gekennzeichnet, dass** das System (301) ferner einen zweiten Katheter (321) umfasst, der Folgendes trägt: einen zweiten Okklusionsballon (311) und einen zweiten volumetrischen Ballon (329) und
wobei der erste Katheter (303) ferner einen dritten Okklusionsballon (312) umfasst, und ferner, wobei der erste Katheter (303) zur Einführung in eine Jugularvene (211) dimensioniert ist und der zweite Katheter (321) zur Einführung in eine Schlüsselbeinvene (219) dimensioniert ist, und
wobei, wenn der erste Katheter (303) und der zweite Katheter (321) so eingeführt sind und der erste, zweite und dritte Okklusionsballon (310, 311, 312) aufgeblasen sind, die Okklusionsballons (310, 311, 312) das Raumvolumen um die Region des Lymphgangs (215) herum sequestrieren, und
wobei, wenn die Okklusionsballons (310, 311, 312) aufgeblasen sind, der erste und zweite volumetrische Ballon (317, 329) aufgeblasen werden können, um Blut aus dem Raumvolumen zu verdrängen.

2. System (301) nach Anspruch 1, wobei, wenn der erste und zweite volumetrische Ballon (317, 329) entleert sind, das Raumvolumen drucklos ist, wodurch Lymphe durch einen Ventilauslass des Lymphgangs (215) gedrängt wird.

3. System (301) nach Anspruch 1, wobei mindestens einer des ersten oder zweiten volumetrischen Ballons (317, 329) dazu konfiguriert ist, auf ein relativ kleineres Volumen zu kollabieren und, wenn derart kollabiert, eine Region mit reduziertem Druck zwischen den Okklusionsballons (310, 311, 312) bewirkt, wobei der reduzierte Druck einen Fluss von Lymphflüssigkeit in die Druckabfallregion verursacht.

4. System (301) nach Anspruch 1, wobei die Okklusionsballons (310, 311, 312) dazu konfiguriert sind, zyklisch aufgeblasen und entleert zu werden.

5. System (301) nach Anspruch 1, wobei, wenn sich das System (301) in einer aufgeblasenen Konfiguration befindet, jeder Ballon (310, 311, 312) sein stromaufwärtiges oder stromabwärtiges Gefäß okkludiert.

6. System (301) nach Anspruch 5, wobei
die stromaufwärtigen Gefäße eine oder mehrere von einer internen Jugularvene, Schlüsselbeinvene oder externen Jugularvene umfassen und
die stromabwärtigen Gefäße die Vena anonyma und/oder die Vena cava superior umfassen.

7. System nach Anspruch 6, ferner umfassend einen Steuerungsmechanismus zum Steuern des Aufblas- und Entleerungszyklus.

## Revendications

1. Système (301) de traitement d'œdème, le système (301) comprenant :
une pluralité de ballonnets d'occlusion (310, 311, 312) conçus pour occlure une pluralité de veines en amont et en aval d'un conduit lymphatique (215) de manière à isoler un volume d'espace dans la zone du conduit lymphatique (215) ;
un premier cathéter (303) raccordé à au moins un premier des ballonnets d'occlusion (310) ;
un premier ballonnet volumétrique (317) disposé le long du premier cathéter (303) et conçu pour occuper une partie substantielle du volume d'espace ; et
au moins une lumière de gonflage servant à gonfler et dégonfler la pluralité de ballonnets (310, 311, 312) ;
**caractérisé en ce que** le système (301) comprend en outre un second cathéter (321), portant : un deuxième ballonnet d'occlusion (311), et un deuxième ballonnet volumétrique (329), et
ledit premier cathéter (303) comprenant en outre un troisième ballonnet d'occlusion (312) et en outre ledit premier cathéter (303) étant dimensionné de manière à être inséré dans une veine jugulaire (211) et ledit second cathéter (321) étant dimensionné de manière à être inséré dans une veine sous-clavière (219), et
lorsque ledit premier cathéter (303) et ledit second cathéter (321) sont ainsi insérés et que les premier, deuxième et troisième ballonnets d'occlusion (310, 311, 312) sont gonflés, lesdits ballonnets d'occlusion (310, 311, 312) séquestrant le volume d'espace autour de la zone du conduit lymphatique (215), et
lorsque lesdits ballonnets d'occlusion (310, 311, 312) sont gonflés, lesdits premier et second ballonnets volumétriques (317, 329) pouvant être gonflés pour déplacer le sang du volume d'espace.

2. Système (301) selon la revendication 1, lorsque lesdits premier et second ballonnets volumétriques (317, 329) sont dégonflés, ledit volume d'espace étant dépressurisé, ce qui pousse la lymphe à travers une sortie de valve du conduit lymphatique (215).

3. Système (301) selon la revendication 1, au moins l'un des premier ou second ballonnets volumétriques (317, 329) étant conçu pour s'affaisser jusqu'à un volume relativement plus petit et, lorsqu'il est ainsi affaissé, réalisant une zone de pression réduite entre lesdits ballonnets d'occlusion (310, 311, 312), ladite pression réduite provoquant un écoulement de fluide lymphatique dans la zone de dépressurisation.

4. Système (301) selon la revendication 1, lesdits ballonnets d'occlusion (310, 311, 312) étant conçus pour être gonflés et dégonflés cycliquement.

5. Système (301) selon la revendication 1, lorsque ledit système (301) se trouve dans une configuration gonflée, chaque ballonnet (310, 311, 312) occluant son vaisseau amont ou aval.

6. Système (301) selon la revendication 5,
lesdits vaisseaux en amont comprenant un ou plusieurs vaisseaux parmi une veine jugulaire interne, une veine sous-clavière ou une veine jugulaire externe, et
lesdits vaisseaux en aval comprenant la veine innommée et/ou la veine cave supérieure.

7. Système selon la revendication 6, comprenant en outre un mécanisme de commande pour commander le cycle de gonflage et de dégonflage.
